# FASCICULE DE BREVET EUROPEEN

(11) **EP 0 594 822 B1**
(45) Date de publication et mention de la délivrance du brevet: **10.09.1997**
(21) Numéro de dépôt: 93910108.5
(22) Date de dépôt: 11.05.1993
(51) Int. Cl.: C12Q 1/68, C12Q 1/70

(54) **Ensemble de moyens pour le dosage quantitatif de rétrovirus, procédé pour sa préparation et kit de diagnostic comprenant ledit ensemble**
Satz Mittel für die quantitative Analyse von Retroviren, Verfahren für dessen Herstellung und denselben enthaltendes Testkit
Set of means for the quantitative analysis of retrovirus, method for preparing the same and diagnosis kit comprising the same

(30) Priorité: 12.05.1992 FR 9205718
(43) Date de publication de la demande: 04.05.1994
(73) Titulaire: Microdiag, 92200 Neuilly, Seine (FR)
(72) Inventeur: ANDRIEU, Jean-Marie, F-75005 Paris (FR); LU, Wei, F-75016 Paris (FR)
(74) Mandataire: Leboyer, Jean-Jacques
(86) Numéro de dépôt international: FR9300455
(87) Numéro de publication internationale: WO9323565

(56) Documents cités:
- WO-A-91/02817
- PROC NATL ACAD SCI U S A 89 (8). 1992. 3241-3245. CODEN: PNASA6 ISSN: 0027-8424 ERON J J ET AL. 'SUSCEPTIBILITY TESTING BY POLYMERASE CHAIN REACTION DNA QUANTITATION A METHOD TO MEASURE DRUG RESISTANCE OF HUMAN IMMUNODEFICIENCY VIRUS TYPE 1 ISOLATES.'
- JOURNAL OF VIROLOGY vol. 66, no. 1, Janvier 1992, pages 334 - 340 W. LU ET AL cité dans la demande
- JOURNAL OF INFECTIOUS DISEASES vol. 162, 1990, CHICAGO US pages 1025 - 1030 J. GENESCA ET AL. cité dans la demande
- NATURE. vol. 349, 10 Janvier 1991, LONDON GB page 111 A. S. BOURINBAIAR 'HIV and gag' cité dans la demande

## Description

La présente invention concerne le diagnostic des maladies dues à des rétrovirus chez l'homme et l'animal, notamment le diagnostic de l'infection par le virus de l'immunodéficience humaine (VIH).

Les rétrovirus font partie de la famille des Retroviridae et sont des virus à ARN qui, grâce à leur transcriptase réverse ou inverse (ci-après en abrégé "TR"), peuvent transcrire leur génome ARN en ADN. Un rétrovirus est ainsi à même de s'introduire dans les cellules qui l'hébergent, sous forme d'ADN chromosomique ou extra-chromosomique. Il en résulte qu'un rétrovirus existe sous deux formes:
- sous forme libre, comme élément génétique mobile à ARN, pouvant passer d'une cellule à une autre;
- sous forme cellulaire rétrotranscrite en ADN, ce qui constitue la forme nécessaire à sa multiplication.

Selon la classification actuelle, la famille des rétrovirus est divisée en trois sous-familles: les Oncovirinae, les Spumavirinae et les Lentivirinae.

Pour plus de clarté, les explications qui suivent et les détails pratiques de l'invention qui sera exposée plus loin sont fournis en référence au rétrovirus VIH, qui fait partie de la famille des Lentivirinae.

Mais ce qui est dit ou démontré à propos de ce virus s'applique mutatis mutandis aux autres rétrovirus. Il est donc entendu que la présente demande de brevet n'est pas limitée au rétrovirus de l'immunodéficience humaine et que c'est simplement dans un souci de simplification de l'exposé et/ou des indications méthodologiques qu'il est principalement fait référence à celui-ci.

L'importance du diagnostic des infections par rétrovirus et la problématique y relative sont résumées ci-après:

La quasi-totalité des personnes séropositives pour le virus de l'immunodéficience humaine (VIH) évolue vers le SIDA dans un délai de 3 à 15 ans après la contamination.

L'évolution du SIDA reste jusqu'à présent fatale dans tous les cas.

Les objectifs actuels de l'homme du métier s'intéressant à la mise au point de remèdes ou de vaccins, de méthodes de traitement et/ou de procédés de diagnostic sont:
- l'évaluation du pronostic de chaque sujet séropositif, à savoir l'appréciation de ce que l'on est en présence chez ce sujet soit d'une évolution rapide vers le SIDA, soit au contraire d'une longue stabilité de l'infection; et
- l'évaluation dans un court délai de l'efficacité d'un médicament chez un sujet séropositif. En effet, jusqu'à présent cette évaluation nécessite un très grand nombre de patients et une longue surveillance, du fait que les critères d'évaluation retenus pour un traitement sont le déclenchement du SIDA ou la mort, événements qui surviennent après des années d'évolution chez un sujet séropositif.

Les travaux de recherche de ces dernières années ont conduit, pour répondre à ces deux objectifs, à chercher à évaluer la quantité de virus elle-même présente dans l'organisme, soit sous forme de virus rétrotranscrit sous forme d'ADN dans les cellules circulantes du sang et particulièrement dans les cellules T4, soit sous forme de virus libre dans le sérum des patients (sous forme d'ARN).

On connaît actuellement deux types de techniques pour mesurer la concentration virale.

Une technique de coculture permet de mesurer in vitro le pourcentage de cellules infectées du sang. Ce pourcentage est faible (inférieur à 1/30 000) chez les personnes séropositives qui resteront stables et asymptomatiques pendant les deux ou trois années suivantes, mais au contraire élevé (supérieur à 1/3000) chez les personnes séropositives qui vont évoluer rapidement vers le SIDA. L'étude de ces pourcentages permet aussi de suivre l'efficacité d'un médicament.

Mais cette technique de culture présente des inconvénients. Elle nécessite en particulier un laboratoire habilité pour des cultures virales actives, dit de type P3. De plus, les manipulations correspondant à la culture peuvent durer jusqu'à 30 jours, ce qui est évidemment trop long pour un examen de laboratoire important, éventuellement utilisable en routine chez de nombreux sujets.

Un deuxième type de technique est la mesure de l'ADN viral des cellules. La mesure de cet ADN se fait par un processus d'amplification physico-chimique utilisant la technique, bien connue de nos jours, de réaction de polymérase en chaîne (couramment dénommée en abrégé "PCR" en référence à l'expression anglaise "Polymerase Chain Reaction"). [Voir par exemple Recent Advances in the Polymerase Chain Reaction par H.A. Erlich et al., Science, vol. 252 (1991), pages 1643-1650]. Compte tenu de la faible reproductibilité de la PCR d'une expérience à l'autre, la mesure doit se faire en présence d'un standard ou étalon, qui est introduit dans chaque expérience sous la forme de plasmides à différentes concentrations contenant chacune une seule copie de l'ADN viral. Selon cette technique (décrite par exemple par J.W. Shih et al. dans The Journal of Infectious Diseases, 1990: 162, pages 1025-1030), en mesurant en même temps l'ADN viral issu de ces différentes concentrations de plasmides et les prélèvements issus de patients, on s'assure que les caractéristiques de la PCR sont identiques au sein d'une même manipulation. Ainsi, au moyen d'une droite de référence obtenue grâce aux concentrations d'ADN viral plasmidique, on peut mesurer l'ADN viral présent dans l'échantillon. La mesure de l'ADN viral grâce à la PCR permet de fixer le pronostic individuel et d'étudier l'efficacité d'un médicament utilisé dans un traitement en cours de la même façon que le pourcentage de cellules infectées. Cette technique demande une expertise en PCR, mais elle ne nécessite pas de laboratoire habilité de type P3 et une trentaine de prélèvements peuvent être traités en 2 ou 3 jours. La coculture aussi bien que la mesure de l'ADN cellulaire ne permettent pas de mesurer la quantité de virus produite dans l'organisme, mais seulement celle du virus rétrotranscrit dans les cellules.

La mesure de la concentration du virus dans le sérum a un intérêt majeur, car il s'agit à travers elle du reflet réel du processus de production du virus par les cellules et du processus de destruction du virus par différents mécanismes, la concentration du virus du sérum étant alors la résultante de ces deux processus.

Il y avait donc un besoin pour parvenir à la détermination de ce type de paramètre et seule la mesure de l'ARN viral le permet; en effet la mesure du pouvoir infectant du plasma n'est qu'un reflet très incomplet de la concentration du virus circulant, compte tenu du fait que chez la quasi-totalité des sujets séropositifs asymptomatiques des anticorps sériques viennent neutraliser l'activité infectieuse du virus.

On a maintenant trouvé de façon inattendue que l'on peut y parvenir en mettant en oeuvre un moyen pour le dosage quantitatif de rétrovirus in vitro comprenant des moyens pour la rétrotranscription en ADN viral de l'ARN viral de prélèvements de liquides et des moyens pour l'amplification de l'ADN viral par réaction de polymérase en chaîne (en abrégé "PCR") et comportant un moyen de quantification des résultats composé d'un étalon exteme formé par des prélèvements de cultures de cellules infectées par des quantités déterminées dudit rétrovirus titrées en leur protéine virale de référence, lesdits prélèvements étant distribués dans du liquide normal dosé en parallèle avec le liquide à tester.

La mesure de l'ARN du virus nécessite quant à elle:
- la rétrotranscription de l'ARN viral en ADN viral, en pratique grâce à l'enzyme appelée transcriptase réverse ou inverse, et
- l'amplification de l'ADN grâce à la PCR mentionnée plus haut.

La deuxième partie de la manipulation est actuellement bien connue et étalonnée comme il est indiqué plus haut.

En revanche, il n'existait jusqu'à présent pas de méthode permettant la standardisation de la première partie de la manipulation, c'est-à-dire le passage de l'ARN viral en ADN viral. Or, le rendement de la transcriptase réverse peut varier d'une manipulation à l'autre d'un facteur allant jusqu'à 2 log (soit un écart de 1 à 100).

L'invention apporte un progrès décisif aux moyens et aux méthodes de dosage quantitatif de rétrovirus in vitro en procurant un ensemble de moyens et une technique simples et efficaces pour la standardisation de ce passage de l'ARN viral en ADN viral.

Comme on l'a indiqué ci-dessus, cet ensemble de moyens comporte un moyen de quantification composé d'un standard externe approprié.

L'invention a donc pour premier objet un ensemble de moyens pour le dosage quantitatif de rétrovirus in vitro, comprenant des moyens pour la rétrotranscription en ADN viral de l'ARN viral de prélèvements de liquide et des moyens pour l'amplification de l'ADN viral par réaction de polymérase en chaîne, caractérisé en ce qu'il comporte un moyen de quantification des résultats composé d'un étalon externe formé par des concentrations virales titrées en leur protéine virale de référence , issues de cultures de cellules infectées par le rétrovirus concerné, lesdites concentrations virales étant distribuées dans un liquide indemne de ce virus et identique au liquide à tester.

L'invention a également pour objet un procédé pour le dosage quantitatif de rétrovirus in vitro, comprenant la rétrotranscription en ADN viral de l'ARN viral de prélèvements de liquide et l'amplification de l'ADN viral par réaction de polymérase en chaîne, caractérisé en ce qu'il comporte, pour la quantification des résultats, la mise en oeuvre d'un étalon externe formé par des prélèvements de cultures de cellules infectées par des quantités déterminées dudit rétrovirus titrées en une protéine de core dudit rétrovirus prise pour référence, lesdits prélèvements étant distribués dans du liquide indemne de ce virus, dosé en parallèle avec le liquide à tester.

La présente invention a en outre pour objet un kit de diagnostic quantitatif comprenant un tel ensemble de moyens, ainsi que l'utilisation de celui-ci pour le diagnostic des maladies à rétrovirus, notamment les virus VIH, les autres lentivirus humains et animaux, les oncovirus humains et animaux et les spumavirus humains et animaux.

Ces objets, ainsi que d'autres qui apparaîtront mieux à la lumière de la description qui suit, seront illustrés plus concrètement en référence aux exemples et aux figures annexées, auxquels il est utile de se référer, mais qui ne limitent en rien l'invention.

Les protéines de référence sont notamment la protéine de core P24/25 pour les virus VIH-1, la protéine P27 pour les virus VIH-2 et les virus SIV, et la protéine P19 pour les virus HTLV-1.

Quant au virus servant à constituer l'étalon externe selon l'invention, on peut en variante lui substituer tout autre rétrovirus ayant sensiblement les mêmes caractéristiques que lui en termes de protéine et d'ARN viral.

On entend par liquide, selon la présente invention, tous les liquides biologiques issus du monde animal ou végétal, comme par exemple le sérum, le plasma, le liquide intracellulaire, la sueur, l'urine, ainsi que l'eau des rivières et autres, mais également les liquides de laboratoire, tels que les surnageants de cultures et autres, et plus généralement toute autre forme de liquide aussi bien naturel qu'élaboré par l'homme.

Pour la mise en oeuvre des moyens selon l'invention, il convient de disposer de concentrations élevées de VIH obtenues par culture de cellules infectées par le VIH. Cela peut être obtenu par une méthode décrite par J.-M. Andrieu et W. Lu dans Journal of Virology, Jan. 1992, pages 334-340.

Sachant que dans les surnageants de cultures virales la concentration en protéine (en l'espèce la protéine P24) non liée au virus ou libre est non significative (inférieure à 1%), la concentration en protéine est proportionnelle au nombre de protéines virales. (cf. A.S. Bourinbaiar, Nature, vol. 349, 10.01.1991).

On a maintenant proposé selon la présente invention d'élaborer un standard externe composé de préférence de concentrations croissantes de virus titrées en P24. On utilise avantageusement pour ce faire des quantités de virus sensiblement équivalentes à 100, 10, 1 et 0,1 pg de P24 distribuées dans environ 0,5 ml de liquide normal qui est soumis aux mêmes manipulations que le liquide à tester.

On a pu observer par ce moyen qu'une concentration d'ARN obtenue par rétrotranscription, puis PCR (mesurée en coups par minute, en abrégé cpm) est parfaitement proportionnelle à la concentration de virus initialement présente (mesurée en P24).

On recommande donc désormais, selon la présente invention, d'utiliser le VIH dont la concentration est mesurée par l'étude de la concentration de la protéine P24 comme standard externe pour le dosage de la concentration d'ARN du virus présent dans le sérum des patients.

Pour s'assurer du caractère parfaitement reproductible de ce standard externe selon l'invention, on a réalisé plusieurs types de manipulations. Tout d'abord des virus provenant respectivement de personnes séropositives et de personnes ayant le SIDA issues de régions géographiques différentes ont été comparés et se sont avérés donner la même concentration d'ARN viral, à condition que les mêmes concentrations de protéines P24 aient été mises en oeuvre.

On a en effet pu démontrer que, quelle que soit l'origine individuelle ou géographique du virus (VIH-1), des concentrations similaires de protéine de core étaient associées à:
1) d'une part un pouvoir infectant semblable de lymphocytes humains (cf. J.-M. Andrieu et W. Lu, op. cité),
2) d'autre part des concentrations sensiblement identiques d'ARN viral.

Par ailleurs on a observé que l'ARN issu de virus inactivés à la chaleur (1 heure à 56°C environ) a exactement les mêmes propriétés que l'ARN issu du virus infectieux: les mêmes concentrations de virus mesurées en protéines P24 sont à l'origine des mêmes concentrations d'ARN viral.

A titre d'exemple concret de mise en oeuvre de ces moyens selon l'invention, on a procédé comme suit:

Un surnageant de culture de HIV-1 infectieux ou inactivé à la chaleur selon les cas, ayant un nombre connu de particules virales (déduit par mesure de la teneur en protéine P24 associée au virion) a été dilué sériellement dans du sérum AB humain (à 1000, 100, 10 et 1 pg/ml). Une fraction aliquote de 500 µl de chaque dilution de surnageant de culture virale ou 500 µl d'échantillons de sérum de patients ont été pastillés par centrifugation et extraits par la méthode RNAzol® B (Wak-Chemie Medical GmbH). Les échantillons d'ARN ont été remis en suspension dans 50 µl d'eau traitée au diéthylpyrocarbonate et contenant 1 mM de DTT (Sigma) et 5 U de RNAsin (Promega). Après traitement avec 2 U de DNase I exempte de RNase (Promega), on a soumis à une rétrotranscription deux parties aliquotes de 10 µl de chaque échantillon d'ARN, et on a amplifié pour un gène gag spécifique de HIV-1 avec un kit RNA PCR (Perkin Elmer Cetus) utilisant des primers (amorces) SK 104/SK 145. On a testé la spécificité de la PCR par amplification simultanée d'ADN de HIV cloné [BH-10 (HTLV-3-B)] et analyse de Southern-blot (transfert d'ADN) pour chaque expérience. On a effectué une PCR en parallèle en l'absence de transcriptase réverse et/ou de modèle de copie (template) comme témoins négatifs. Deux parties aliquotes de 10 µl de chacun des produits de PCR amplifiés ont été directement transférées sur une membrane en Nylon et testées avec un SK 19 marqué aux extrémités au gamma-³²P. On a quantifié le signal du produit de PCR en déterminant la valeur de radioactivité en cpm (coups par minute). La courbe standard (étalon) de particules virales d'ARN pour chaque lot d'expériences a été déterminée à partir de la moyenne et de l'écart-type d'une itération de quatre points de chacune des dilutions. On a procédé aux évaluations des échantillons de sérum des patients par une formule dérivée de la courbe standard. On a ainsi pu vérifier que les droites standards obtenues au cours de manipulations différentes restaient toujours parallèles aux droites standards témoins de la PCR d'ADN et servaient de témoins de sensibilité et de reproductibilité de la PCR.

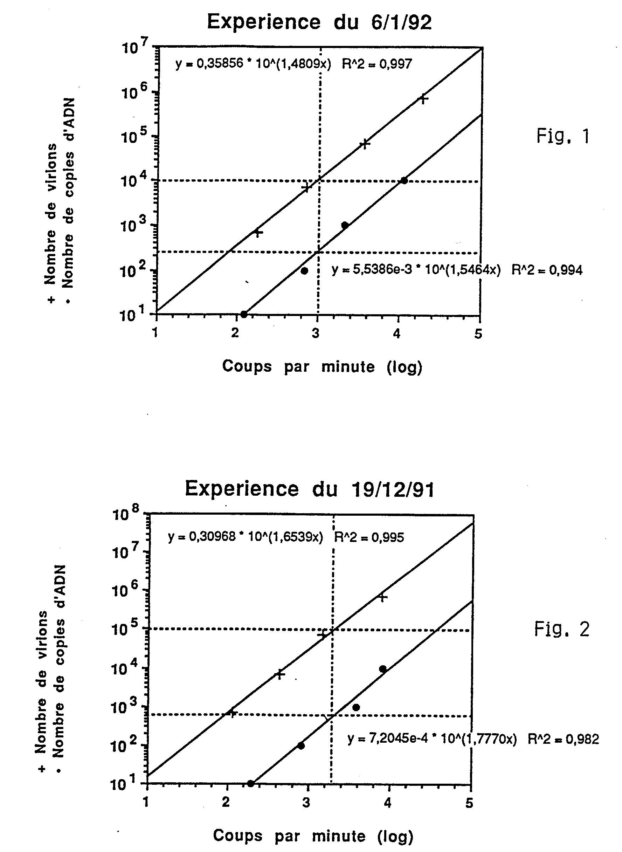

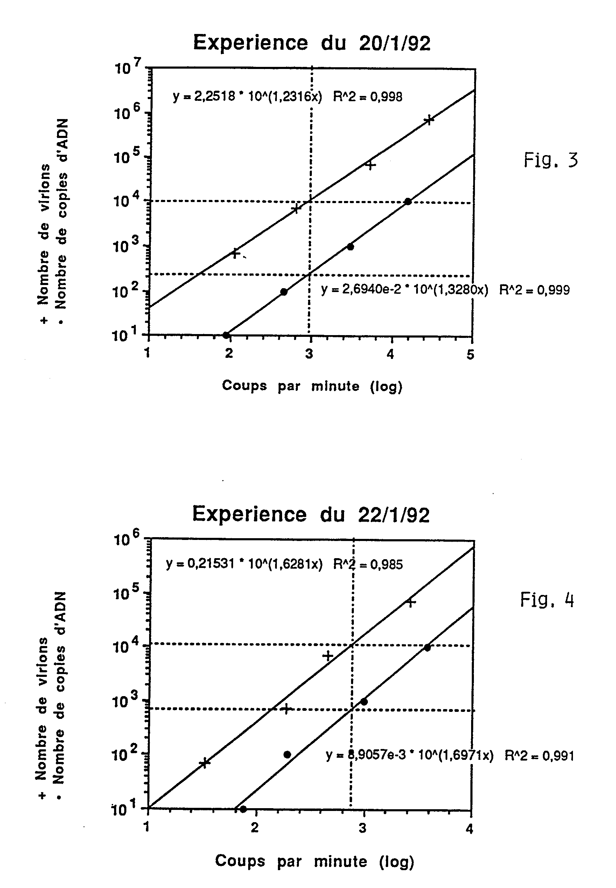

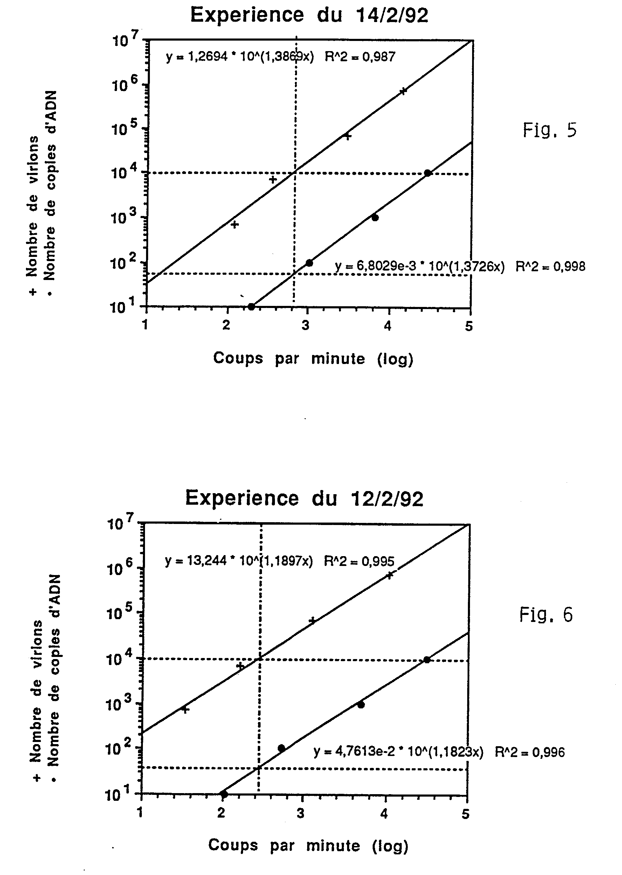

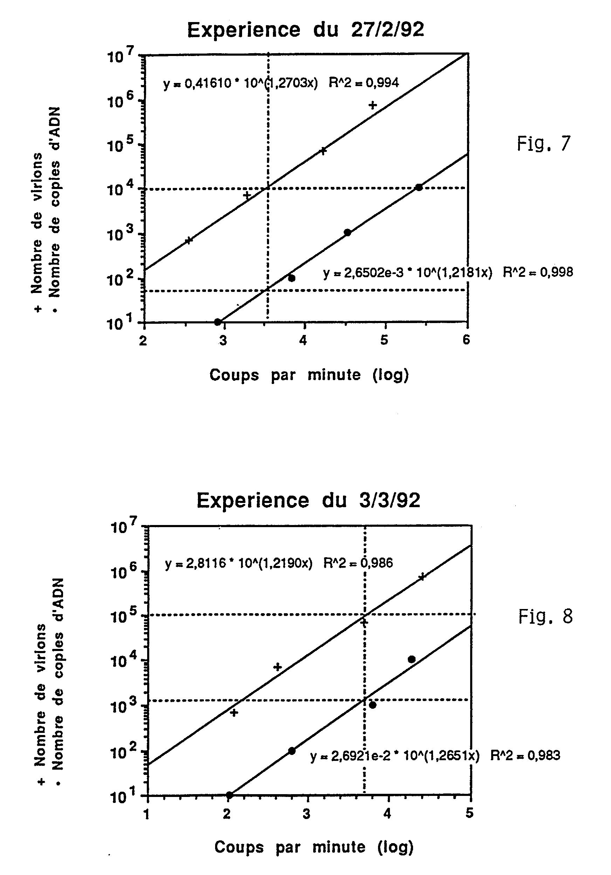

Les résultats obtenus dans des expériences conduites conformément à cette description sont représentés sur les fig. 1 à 8 annexées.

Ces figures sont des graphiques où sont portés respectivement en abscisse le log du nombre de coups par minute (cpm) comptés au compteur Geiger comme quantification du signal du produit de PCR et en ordonnée le nombre de copies ARN/ADN.

La droite de référence du bas est le standard ADN (pour différentes concentrations de plasmide viral). La droite de référence résultant du standard externe selon l'invention est dans tous les cas pratiquement parallèle au standard ADN, et surtout son écart avec la courbe étalon est variable d'une manipulation à l'autre, ce qui confirme sans conteste la nécessité de l'étalon selon la présente invention. Cela est lié à une activité de transcriptase réverse similaire au sein d'une même manipulation, mais différente d'une manipulation à l'autre.

On vérifie ainsi que pour les dilutions de virus standards, le ratio de rétrotranscription est sensiblement constant au sein d'une même manipulation.

D'autres éléments relatifs aux résultats de ces essais comparatifs pour lesquels ont été mis en oeuvre les moyens selon l'invention se trouvent dans les tableaux 1 et 2 ci-dessous.

Ainsi la présente invention permet d'établir qu'un standard externe issu d'un pool de virus ou de souches virales équivalentes, de préférence inactivés par la chaleur ou tout moyen équivalent, provenant d'un grand nombre de patients infectés par le rétrovirus concerné, peut être utilisé par tout laboratoire qui souhaite mesurer la concentration du dit virus.

En pratique on recommande d'utiliser quatre concentrations, de préférence de virus inactivé, distribuées dans du liquide normal auquel on fait subir l'ensemble des manipulations de la même façon qu'au liquide testé, pour obtenir une droite de référence.

II convient dans la pratique d'établir ce standard lors de chaque manipulation.

**TABLEAU 1 -**

| Quantification des particules virales (vp) de HIV-1 du sérum de patients par l'ARN(TR)-PCR en utilisant la souche virale avec un nombre connu de vp de HIV-1 comme standard externe. | | | |
|---|---|---|---|
| Stades des patients selon CDC | cellules CD4 T circulantes/µl | Moyenne ± écart-type de vp/variation intra-test (log max-log min) | Moyenne ± écart-type de vp/variation inter-test (log max-log min) |
| | | (1) | (2) |
| CDC II (asymptomatique) | 934 | 469 ± 85 / (0,282) | 481 ± 212 / (0,532) |
| CDC III (asymptomatique) | 674 | 4021 ± 624 / (0,253) | 3788 ± 1535 / (0,471) |
| CDC IVc (SK) | 380 | 377946 ± 71459 / (0,279) | 410673 ± 125556 / (0,538) |
| CDC IVb (IO) | 34 | 1559378 ± 265742 / (0,322) | 1368712 ± 436657 / (0,526) |

| | | | |
|---|---|---|---|
| (1): Les échantillons de sérum ont été testés cinq fois dans une expérience | | | |
| (2): Les échantillons de sérum ont été testés sur cinq lots différents dans l'expérience | | | |

Dans le tableau 1 ci-dessus:
- CDC =: Centers of Disease Control (Atlanta, USA)
- SK =: Sarcome de Kaposi
- IO =: Infection opportuniste

Le tableau 1 montre la similitude de virus provenant de patients très différents et la variation intra-test et inter-tests.

**TABLEAU 2 -**

| Amplification par ARN(TR)-PCR de 100 pg de souches de HIV-1 infectieuses ou inactivées par la chaleur, isolées à partir de patients asymptomatiques ou ayant le SIDA . | | | |
|---|---|---|---|
| Stades des patients selon CDC | cellules CD4 T circulantes/µl | Mesure quantitative d'amplification par transcriptase réverse, puis PCR | |
| | | VIH infectieux (moyenne ± écart-type de cpm) | VIH inactivé à la chaleur (moyenne ± écart-type de cpm) |
| CDC II (asymptomatique) | 934 | 15441 ± 2046 | 17646 ± 299 |
| | | | |
| CDC III (asymptomatique) | 674 | 15110 ± 2170 | 15511 ± 2108 |
| | | | |
| CDC IVc (SK) | 380 | 15756 ± 2867 | 10479 ± 1202 |
| | | | |
| CDC IVb (IO) | 34 | 17019 ± 2757 | 16988 ± 3007 |
| * Cela correspond à 10⁶ particules virales | | | |
| ** Les échantillons de sérum ont été testés cinq fois dans une expérience | | | |

## Revendications

1. Ensemble de moyens pour le dosage quantitatif de rétrovirus in vitro, comprenant des moyens pour la rétrotranscription en ADN viral de l'ARN viral de prélèvements de liquide et des moyens pour l'amplification de l'ADN viral par réaction de polymérase en chaîne (en abrégé "PCR"), caractérisé en ce qu'il comporte un moyen de quantification des résultats composé d'un étalon externe formé par des concentrations virales titrées en leur protéine virale de référence, issues de cultures de cellules infectées par le rétrovirus concerné, lesdites concentrations virales étant distribuées dans un liquide indemne de ce virus et identique au liquide à tester.

2. Ensemble de moyens selon la revendication 1, caractérisé en ce que le rétrovirus est choisi parmi les rétrovirus VIH-1; VIH-2, HTLV-1, HTLV-2 et SIV.

3. Ensemble de moyens selon la revendication 1, caractérisé en ce que le liquide est un sérum animal ou humain.

4. Ensemble de moyens selon la revendication 1, caractérisé en ce que le rétrovirus est du virus VIH-1 ou VIH-2 et la protéine de référence est une protéine P24/25 ou P27.

5. Ensemble de moyens selon l'une quelconque des revendications 1 à 4, caractérisé en ce que lesdites quantités déterminées de rétrovirus sont des quantités équivalentes respectivement à 100, 10, 1 et 0,1 pg de protéine prise pour référence distribuées dans environ 0,5 ml de liquide indemne du virus concerné.

6. Kit de diagnostic pour le dosage des rétrovirus, caractérisé en ce qu'il comprend un ensemble de moyens selon l'une quelconque des revendications 1 à 5.

7. Procédé pour le dosage quantitatif de rétrovirus in vitro, comprenant la rétrotranscription en ADN viral de l'ARN viral de prélèvements de liquide et l'amplification de l'ADN viral par réaction de polymérase en chaîne, caractérisé en ce qu'il comporte, pour la quantification des résultats, la mise en oeuvre d'un étalon externe formé par des prélèvements de cultures de cellules infectées par des quantités déterminées dudit rétrovirus, titrées en une protéine de core dudit rétrovirus prise pour référence, lesdits prélèvements étant distribués dans du liquide indemne de ce virus, dosé en parallèle avec le liquide à tester.

8. Procédé selon la revendication 7, caractérisé en ce que le rétrovirus est choisi parmi les rétrovirus VIH-1, VIH-2, HTLV-1, HTLV-2 et SIV, d'origine animale ou humaine.

9. Procédé selon l'une des revendications 7 ou 8, caractérisé en ce que lesdites quantités déterminées de rétrovirus sont des quantités équivalentes respectivement à 100, 10, 1 et 0,1 pg de protéine de référence distribuées dans environ 0,5 ml de liquide normal.

10. Utilisation d'un ensemble de moyens selon l'une quelconque des revendications 1 à 5 pour le diagnostic des maladies à rétrovirus, notamment les virus VIH, les autres lentivirus humains et animaux, les oncovirus humains et animaux et les spumavirus humains et animaux.

## Patentansprüche

1. Kombination von Mitteln zur quantitativen Retrovirus-Bestimmung *in vitro,* umfassend Mittel zur reversen Transkription der viralen RNA aus Flüssigkeitsproben in virale DNA und Mittel zur Amplifikation der viralen DNA durch Polymerase-Ketten-Reaktion (abgekürzt: "PCR"), dadurch gekennzeichnet, daß die Kombination ein Mittel zur Quantifizierung der Ergebnisse enthält, welches einen externen Standard umfaßt, gebildet aus Viruskonzentrationen, die nach ihrem viralen Referenzprotein titriert wurden, erhalten aus mit dem betreffenden Retrovirus infizierten Zellkulturen, wobei diese Viruskonzentrationen in einer Virus-freien und mit der Testflüssigkeit identischen Flüssigkeit verteilt sind.

2. Kombination von Mitteln nach Anspruch 1, dadurch gekennzeichnet, daß das Retrovirus ausgewählt ist aus der Gruppe bestehend aus HIV-1, HIV-2, HTLV-1, HTLV-2 und SIV.

3. Kombination von Mitteln nach Anspruch 1, dadurch gekennzeichnet, daß die Flüssigkeit tierisches oder menschliches Serum ist.

4. Kombination von Mitteln nach Anspruch 1, dadurch gekennzeichnet, daß das Retrovirus HIV-1 oder HIV-2 ist und das Referenzprotein ein Protein P24/25 oder P27 ist.

5. Kombination von Mitteln nach einem der Ansprüche 1 bis 4, dadurch gekennzeichnet, daß die vorgegebenen Retrovirus-Mengen 100, 10, 1 bzw. 0,1 pg Referenzprotein entsprechen, verteilt in ungefähr 0,5 ml Flüssigkeit, die frei ist von dem betreffenden Virus.

6. Diagnostischer Kit zur Retrovirus-Bestimmung, dadurch gekennzeichnet, daß er eine Kombination von Mitteln nach einem der Ansprüche 1 bis 5 umfaßt.

7. Verfahren zur quantitativen Retrovirus-Bestimmung *in vitro* umfassend die reverse Transkription in virale DNA von viraler RNA aus Flüssigkeitsproben und die Amplifikation von viraler DNA durch Polymerase-Ketten-Reaktion, dadurch gekennzeichnet, daß man zur Quantifizierung der Ergebnisse einen externen Standard aus Proben von mit vorgegebenen Mengen des betreffenden Retrovirus infizierten Zellkulturen verwendet, die nach einem Core-Protein des betreffenden Retrovirus titriert wurden, wobei diese Proben in einer des betreffenden Virus-freien Flüssigkeit verteilt sind, parallel dosiert zur zu testenden Flüssigkeit.

8. Verfahren nach Anspruch 7, dadurch gekennzeichnet, daß das Retrovirus ausgewählt ist aus der Gruppe bestehend aus HIV-1, HIV-2, HTLV-1, HTLV-2 und SIV, tierischer oder menschlicher Herkunft.

9. Verfahren nach Anspruch 7 oder 8, dadurch gekennzeichnet, daß die vorgegebenen Retrovirus-Mengen 100, 10, 1 bzw. 0,1 pg Referenzprotein entsprechen, verteilt in ungefähr 0,5 ml normaler Flüssigkeit.

10. Verwendung einer Kombination von Mitteln nach einem der Ansprüche 1 bis 5 zur Diagnose retroviraler Krankheiten, insbesondere der HIV-Viren, der anderen menschlichen und tierischen Lentiviren, der menschlichen und tierischen Onkoviren und der menschlichen und tierischen Spumaviren.

## Claims

1. A combination of means for the quantitative determination of retrovirus in vitro, comprising means for the retrotranscription in viral DNA of viral RNA of samples of liquid and means for amplifying the viral DNA by polymerase chain reaction (abbreviated "PCR"), characterized in that it comprises a means for quantifying the results which is composed of an external standard formed by viral concentrations standardized in their viral reference protein, obtained from cultures of cells infected by the retrovirus concerned, said viral concentrations being distributed in a liquid free of said virus and identical to the liquid to be tested.

2. A combination of means according to Claim 1, characterized in that the retrovirus is selected from among the retroviruses HIV-1, HIV-2, HTLV-1, HTLV-2 and SIV.

3. A combination of means according to Claim 1, characterized in that the liquid is an animal of human serum.

4. A combination of means according to Claim 1, characterized in that the retrovirus is HIV-1 or HIV-2 virus, and the reference protein is a P24/25 or P27 protein.

5. A combination of means according to any of Claims 1 to 4, characterized in that the said determined quantities of retrovirus are quantities equivalent to 100, 10, 1 and 0.1 pg respectively of reference protein, which are distributed in about 0.5 ml of liquid free of the virus concerned.

6. A diagnostic kit for the determination of retroviruses, characterized in that it comprises a combination of means according to anyone of Claims 1 to 5.

7. A method for the quantitative determination of retrovirus in vitro, comprising the retrotranscription in viral DNA of viral RNA of samples of liquid and the amplification of the viral DNA by polymerase chain reaction, characterized in that it comprises, for the quantification of the results, the use of an external standard formed by samples of cultures of cells infected by given quantities of said retrovirus, titrated in a core protein of said retrovirus used as a reference, said samples being distributed in liquid free of said virus, determined in parallel with the liquid to be tested.

8. A method according to Claim 7, characterized in that the retrovirus is selected from among the retroviruses HIV-1, HIV-2, HTLV-1, HTLV-2 and SIV, of animal or human origin.

9. A method according to either of Claims 7 and 8, characterized in that said determined quantities of retrovirus are quantities equivalent to 100, 10, 1 and 0.1 pg respectively of reference protein, which are distributed in about 0.5 ml of standard liquid.

10. The use of a means according to anyone of Claims 1 to 5, for the diagnosis of retrovirus disease, in particular HIV viruses, the other human and animal lentiviruses, the human and animal oncoviruses, and the human and animal spumaviruses.
